# DEMANDE DE BREVET EUROPEEN

(11) **EP 0 580 465 A1**
(43) Date de publication de la demande: **26.01.1994**
(21) Numéro de dépôt: 93401620.5
(22) Date de dépôt: 24.06.1993
(51) Int. Cl.: A61K 31/495, A61K 31/505, C07D 213/74

(54) **Nouvelle utilisation thérapeutique d'hétérocyclyl-pipérazines comme 5-HT3 agonistes et nouveaux dérivés**

(30) Priorité: 25.06.1992 EP 92401800
(71) Demandeur: SANOFI, F-75008 Paris (FR); MIDY S.p.A., I-20137 Milano (IT)
(72) Inventeur: Baroni, Marco, I-20010 Vanzago (Milano) (IT); Guzzi, Umberto, I-20100 Milano (IT); Giudice, Antonina, I-20154 Milano (IT); Landi, Marco, I-Bussero (Milano) (IT)
(74) Mandataire: Le Roux, Martine

(57) **Abrégé**

Nouvelle utilisation thérapeutique d'hétérocyclyl-pipérazines de formule générale (I) 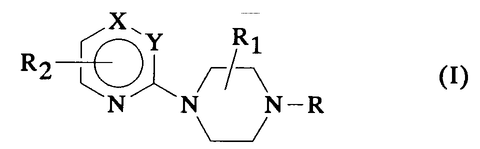
dans laquelle R représente l'hydrogène ou un groupe (C₁-C₄)alkyle, R₁ représente l'hydrogène ou un groupe méthyle, R₂ représente un hydrogène ou un halogène; X et Y représentent chacun -CH=, ou l'un est -CH= et l'autre -N=, ou un de leur sels pharmaceutiquement acceptables pour la préparation de médicaments destinés au traitement et à la prophylaxie des états pathologiques qui peuvent être améliorés à l'aide d'une action agoniste médiée par les récepteurs du type 5-HT₃ et nouveaux composés dérivés de cette structure.

## Description

La présente invention concerne une nouvelle utilisation thérapeutique d'une classe d'hétérocyclyl-pipérazines et de nouveaux produits dérivant de cette structure.

Un premier objet de la présente invention est une nouvelle utilisation de composés de formule générale (I) 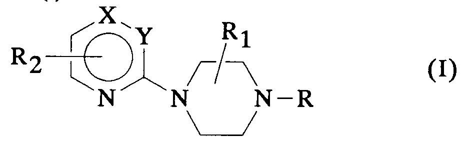
dans laquelle R représente un hydrogène ou un groupe (C₁-C₄)alkyle; R₁ représente un hydrogène ou un groupe méthyle; R₂ représente un hydrogène ou un halogène; X et Y représentent chacun -CH=, ou l'un est -CH= et l'autre -N=; ainsi que de leurs sels pharmaceutiquement acceptables.

Les brevets US 4,078,063 et EP 65 757 décrivent des 2-pipérazinyl-pyridines dans lesquelles la pyridine est substituée par un halogène; ces produits ayant une activité anorexigène, antidépressive, antihypertensive.Les brevets US 4,163,849 et BE 840904 décrivent des 6-chloro-2-pipérazinyl-pyrazines dans lesquelles la pipérazine peut être substituée par un groupe alkyle en position 4; ces produits aussi ont montré une activité anorexigène.Dans la publication, Eur. J. Pharmacol. 168, N°3, 387-392, 1989, on décrit des études de binding 5-HT₃ sur certaines arylpipérazines, mais aucune indication est donnée sur l'éventuelle activité agoniste ou antagoniste de ces produits.

Des 2-pipérazinyl-pyrimidines sont décrites dans le brevet DE 3507983 en tant qu'intermédiaires de synthèse.

On a maintenant trouvé que les produits de formule (I) présentent une activité sur le système sérotoninergique par médiation par les récepteurs 5-HT₃ dont ces composés sont de puissants agonistes.

La présente invention concerne donc, selon un de ses aspects, l'utilisation d'un composé de formule (I) pour la préparation de médicaments destinés à la prophylaxie et au traitement des pathologies liées au système sérotoninergique, plus particulièrement à la prophylaxie et au traitement des troubles du système sérotoninergique lorsque l'on souhaite avoir une action agoniste sélective par médiation par les récepteurs 5-HT₃.

Dans la présente description, le terme (C₁-C₄)alkyle désigne le résidu d'un hydrocarbure aliphatique saturé à chaîne droite ou ramifiée qui contient un à quatre atomes de carbone, tel que méthyle, éthyle, propyle, isopropyle, n-butyle, isobutyle, sec-butyle, t-butyle; le terme halogène désigne l'un des quatre halogènes communs, de préférence le chlore, le fluore et le brome; le noyau contenant X et Y (dont la signification est donnée ci-dessus) constitue une pyridine, une pyrazine ou une pyrimidine.

Les sels d'addition des composés de formule (I), avec les acides physiologiquement compatibles, (plus précisemment leur utilisation) font également partie de la présente invention.

Les sels physiologiquement compatibles comprennent les sels d'addition avec des acides minéraux ou organiques physiologiquement compatibles, tels que les chlorhydrates, les bromhydrates, les sulfates, les phosphates, les acétates, les tartrates, les succinates, les maléates, les fumarates, les mésylates, les tosylates etc..

Les composés de formule (I) peuvent aisément être préparés selon une méthode générale qui comporte la réaction entre une pipérazine de formule (II) 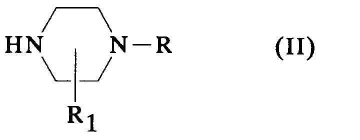
et un hétérocycle aromatique de formule (III) 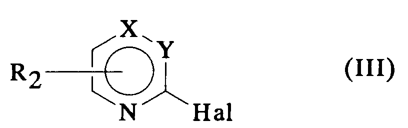
dans lesquelles Hal représente un atome d'halogène et R, R₁, R₂, X et Y sont tels que définis précédemment.

La réaction est généralement conduite en mélangeant les composés (II) et (III) dans un solvant convenable et en chauffant à une température compnse entre la température ambiante et 200°C, selon le solvant utilisé et le temps de réaction.

Cette synthèse a été décrite en détail dans les brevets US 4,078,063, EP 65757, US 4,163,849, BE 840904 et DE 3507983 mentionnés ci-dessus.

L'affinité des composés de formule (I) pour les récepteurs 5-HT₃, a été mise en évidence à l'aide des essais de binding in vitro en utilisant les sites de liaison 5-HT₃ présents dans le cortex cérébral du rat (G.J. Kilpatrick, B.J. Jones et M.B. Tyers. Identification and distribution of 5-HT₃ receptors in rat bran using radioligand binding. *Nature, 1987;* ***330:*** *746-8)* et comme ligand marqué le [³H] BRL 43694 (granisetron), un antagoniste des récepteurs 5-HT₃ puissant et spécifique.

La préparation des membranes et le test de binding ont été effectués selon la méthode décrite par Nelson et Thomas (D.R. Nelson et D.R. Thomas. [³H] BRL 43694 (granisetron), a specific ligand for 5HT₃ binding sites in rat brain cortical membranes. *Biochem. Pharmacol., 1989;* ***38:*** *1693-5).*

Les résultats ont été évalués avec des méthodes d'ajustement ("fitting") non linéaire "Accufit saturation", pour les études de saturation (H.A. Feldman. Mathematical theory of complex ligand-binding systems at equilibrium: some methods of parameter fitting. *Analyt. Biochem., 1972;* ***48:*** *317-38)* et "Accufit competition", pour les études de déplacement (H.A. Feldman, D. Rodbard et D. Levine. Mathematical theory of cross reactive radioimmunoassay and ligand-binding systems at equilibria. *Analyt. Biochem., 1972;* ***45:*** *530-56).*

Afin d'évaluer les affinités des composés, dans les études de compétition on a utilisé une concentration de 0,5 nM de [³H] BRL 43694.

Les composés de formule (I) se sont montrés en général plus puissants que la sérotonine et la 2-méthyl-sérotonine dans le déplacement du [³H] BRL 43694.

On a confirmé l'affinité et mis en évidence l'activité agoniste au niveau des récepteurs 5-HT₃ grâce aussi à des études chez le rat anesthésié, en particulier en administrant les composés par voie intraveineuse et en observant la diminution fugace de la fréquence cardiaque (effet Bezold-Jarisch), dont l'intensité varie selon la dose, comparable à celle obtenue en administrant de la sérotonine ou de la méthyl-sérotonine.

Cet effet est inhibé par les antagonistes sélectifs des récepteurs 5-HT3 (par exemple ICS 205930 et zacopride), tandis qu'il n'est pas inhibé par les antagonistes des récepteurs D de la sérotonine (par exemple méthysergide).

Plus particulièrement, l'effet Bezold-Jarisch provoqué par les composés de formule (I) a été évalué en utilisant des rats Sprague-Dawley d'un poids compris entre 200 et 300 g, anesthésiés par une dose intrapéritonéale de 1,25 g/kg d'uréthane. La pression artérielle est enregistrée au niveau d'une artère carotide et la fréquence cardiaque évaluée par la fréquence des pulsations à l'aide d'un cardiotachymètre. Un cathéter est placé dans la veine jugulaire pour l'administration des substances.

Des doses différentes des composés à tester sont administrées par voie intraveineuse dans un volume de 0,5 ml/kg.

La bradycardie provoquée par chaque dose est exprimée comme inhibition en pourcentage de la fréquence basale. On peut ainsi calculer la DE₅₀, c'est-à-dire la dose qui diminue de 50% la fréquence cardiaque chez les animaux traités.

Les composés les plus intéressants ont montré des résultats, exprimés en DE₅₀, qui sont compris entre 0,5 et 10 µg/kg.

En ce qui concerne cette activité agoniste des récepteurs 5-HT₃, la 6-chloro-2-pipérazinylpyridine, la 6-chloro-2-(4-méthylpipérazinyl)pyridine, la 6-bromo-2-(4-méthylpipérazinyl)pyridine et la 6-chloro-2-(3-méthylpipérazinyl)pyridine et leurs sels pharmaceutiquement acceptables sont des composés particulièrement intéressants.

Grâce à leur puissante activité agoniste vis-à-vis des récepteurs 5-HT₃ et à leur très faible toxicité, les produits de formule (I) peuvent être utilisés comme médicaments dans le traitement des troubles qui impliquent le système sérotoninergique tant périphérique, que central.

On peut par exemple envisager l'emploi thérapeutique de ces produits dans le traitement des troubles dysthymiques ou bien dans le cas d'anxiété ou de troubles psychotiques.

On peut également envisager l'utilisation de ces produits dans le traitement des désordres de la motricité intestinale et en particulier dans le traitement de la constipation.

L'activité anticonstipante a été démontrée en soumettant les composés de formule (I) à un test sur l'étude de l'élimination fécale chez le rat; cet essai a été conduit selon la méthode expérimentale décrite dans la demande de brevet EP 412901.

Les résultats du test sont exprimés en [IA-1g], qui représente l'index d'activité indiquant la dose active qui stimule l'élimination d'1 g de selles (poids sec).

Les composés de formule (I) se sont montrés très actifs dans ce test, en donnant des résultats en [IA-1g] compris dans un intervalle de 0,5 à 3 mg/kg.

Pour leur utilisation thérapeutique, les produits de formule (I), purs ou en association avec toute autre substance pharmaceutiquement compatible, peuvent être utilisés sous des formes pharmaceutiques convenables, destinées à l'administration par voie orale et parentérale, sublinguale, rectale, transdermale.

Pour l'administration orale on peut par exemple utiliser des comprimés, des dragées, des granules ou des compositions liquides, telles que des sirops, des émulsions, des solutions.

Pour l'administration parentérale on peut utiliser des compositions stériles injectables, aqueuses ou non aqueuses, des suppositoires pour l'administration rectale, des patches pour l'administration transdermique; le cas échéant on peut préparer des formes retard ou des formes dans lesquelles le principe actif de formule (I) est inclus dans des lyposomes ou dans des cyclodextrines.

Ces formes pharmaceutiques sont préparées selon les méthodes conventionnelles, en mélangeant les principes actifs de formule (I) avec les excipients et les additifs habituellement employés dans la technique pharmaceutique, tels que l'amidon, le lactose, le talc, le stéarate de magnésium, le saccharose, l'huile de paraffine, les produits mouillants, aromatisants, stabilisants, etc.

Le dosage approprié dans l'emploi thérapeutique de ces compositions doit être évalué selon le cas, en considérant les caractéristiques des sujets à traiter : l'âge, le poids du corps, etc., ainsi que la gravité des affections à traiter; en général cette nouvelle utilisation thérapeutique prévoit l'administration d'un dosage compris entre 0,05 et 100 mg/jour, préférablement entre 0,1 et 50 mg, éventuellement subdivisé en doses à administrer plusieurs fois par jour, la posologie préférée étant une à trois fois par jour.

Comme exemple, on peut préparer des comprimés ayant la composition suivante:

| | |
|---|---|
| 6-bromo-2-(4-méthylpipérazinyl)pyridine | 5 mg |
| lactose | 50 mg |
| talc | 44 mg |
| stéarate de magnésium | 1 mg |

La présente invention concerne également de nouvelles hétérocyclylpipérazines de formule (I):
- la 6-chloro-2-(3-méthylpipérazinyl)pyridine et ses sels d'addition pharmaceutiquement acceptables;
- la 6-chloro-2-(4-méthylpipérazinyl)pyridine et ses sels d'addition pharmaceutiquement acceptables;
- la 6-bromo-2-(4-méthylpipérazinyl)pyridine et ses sels d'addition pharmaceutiquement acceptables.

La préparation de ces nouvelles hétérocyclylpipérazines est une synthèse telle que décrite plus avant dans le présent texte. Elle est illustrée par les exemples ci-après.

La présente invention concerne enfin les compositions pharmaceutiques contenant en tant que principe actif au moins une desdites nouvelles hétérocyclylpipérazines ci-dessus, sous forme libre ou de sel d'addition avec un acide physiologiquement compatible.

### EXEMPLE 1

### Chlorhydrate de 6-chloro-2-(4-méthylpipérazinyl)pyridine

A une solution de 7,5 g (0,05 mole) de 2,6-dichloropyridine dans 70 ml de DMSO, on ajoute 6,9 g (0,05 mole) de carbonate de potassium anhydre et 5,5 ml (0,05 mole) de N-méthylpipérazine. On chauffe à 80°C pendant 4 heures; on verse dans un mélange eau/glace, extrait à l'acétate d'éthyle, sèche sur Na₂SO₄, filtre et évapore à sec. On reprend le résidu dans 30 ml d'isopropanol et on prépare le chlorhydrate à l'aide d'isopropanol chlorhydrique. On filtre le précipité, le recrystallise dans de l'isopropanol et on obtient ainsi 4,8 g de poudre blanche . P.f. 225-227°C.
Pour mieux purifier le produit, on le dissout dans de l'eau rendue basique par NaOH, on extrait à l'acétate d'éthyle, on lave la phase organique à l'eau, on agite pendant 10 minutes avec du gel de silice, on sèche sur Na₂SO₄, filtre et évapore à sec.
On reprépare le chlorhydrate à l'aide d'isopropanol saturé avec de l'acide chlorhydrique gazeux en obtenant 1,5 g du composé du titre. P.f. 233-235°C.

### EXEMPLE 2

### Chlorhydrate de 6-bromo-2-(4-méthylpipérazinyl)pyridine

On mélange 11,84 g (0,05 mole) de 2,6-dibromopyridine, 70 ml de DMSO, 6,9 g (0,05 mole) de K₂CO₃ anhydre et 5,5 ml (0,05 mole) de N-méthylpipérazine. On chauffe à 80°C pendant 4 heures; on verse dans un mélange eau/glace, on extrait à l'acétate d'éthyle, on sèche sur Na₂SO₄, on filtre et l'on évapore à sec. On reprend le résidu dans 30 ml d'éther éthylique et on agite pendant 10 minutes avec du gel de silice; on sèche sur Na₂SO₄, on filtre et l'on évapore à sec. On prépare le chlorhydrate à l'aide d'isopropanol saturé avec de l'acide chlorhydrique gazeux, on obtient ainsi 8,7 g de produit qui, après cristallisation dans de l'éthanol 95%, donnent 7 g du composé du titre. P.f. 273-275°C.

### EXEMPLE 3

### Chlorhydrate de 6-chloro-2-(3-méthylpipérazinyl)pyridine

(a) **1-benzyl-3-méthylpipérazine**
   On mélange en refroidissant 126,5 g (1 mole) de chlorure de benzyle, 100 g (1 mole) de 2-méthylpipérazine, 138 g (1 mole) de K₂CO₃ (poudre anhydre) et 2 g de NaI dans 500 ml de méthyléthylcétone. On chauffe au reflux pendant 2 heures; on laisse refroidir le mélange, on filtre le K₂CO₃ et on sèche. On reprend le résidu dans de l'eau alcalinisée par NaOH, on extrait avec de l'éther éthylique, on lave à l'eau, on sèche sur Na₂SO₄ et l'on évapore à sec. On distille et l'on obtient 55 g du produit composé du titre. (P.e. 140-150°C/20-25 mmHg).
(b) **1-benzyl-4-formyl-3-méthylpipérazine**
   A 54 g (0,283 mole) du produit de l'étape a), on ajoute 10,7 ml (0,283 mole) d'acide formique (99%). On chauffe à 180°C pendant 4 heures, puis on distille (p.e. 145°C/0,05 mmHg) et on obtient 52,5 g de 1-benzyl-4-formyl-3-méthylpipérazine.
(c) **1-formyl-2-méthylpipérazine**
   On dissout 51 g (0,233 mole) du produit de l'étape b) dans 300 ml d'alcool éthylique absolu et on ajoute 6 g de Pd/C 10% à la solution ainsi obtenue. On hydrogène à la pression atmosphérique en chauffant jusqu'à 35-40°C. La réaction peut être suivie par CCM et, une fois terminée, après 6 heures environ, on sépare le catalyseur par filtration et on sèche. On obtient ainsi une huile qui est distillée (p.e. 95°C/0,3 mmHg). Rendement: 23 g du produit du titre.
(d) **6-chloro-2-(4-formyl-3-méthylpipérazinyl)pyridine**
   On mélange 20,5 g (0,138 mole) de 2,6-dichloropyridine, 19,1 g (0,138 mole) de K₂CO₃, 21,5 g (0,155 mole) du produit de l'étape c) dans 140 ml de xylène. On chauffe au reflux pendant 14 heures, on laisse refroidir le mélange et on y ajoute de l'éther éthylique. On lave à l'eau et ensuite à l'HCl dilué, on sèche sur Na₂SO₄, on filtre et l'on évapore le solvant. On reprend le résidu dans de l'éther éthylique, on extrait à l'HCI 2N. On sépare les deux phases et on alcalinise la phase aqueuse. On extrait à l'éther éthylique, on lave à l'eau et on sèche sur Na₂SO₄. On filtre et l'on évapore le solvant. On obtient ainsi 14 g de 6-chloro-2-(4-formyl-3-méthylpipérazinyl) pyridine.
e) **Chlorhydrate de 6-chloro-2-(3-méthylpipérazine)pyridine**
   On chauffe au reflux 14 g (0,0584 mole) du produit de l'étape d) dans 200 ml de HCl 2N pendant 1 heure. On laisse refroidir, on lave à l'éther éthylique, on alcalinise à l'aide de NaOH dilué, on extrait à l'éther éthylique et on sèche sur Na₂SO₄. On filtre et on prépare le chlorhydrate à l'aide d'isopropanol saturé avec de l'acide chlorhydrique gazeux. On filtre et on recristallise dans de l'isopropanol. On obtient ainsi 8 g de chlorhydrate de 6-chloro-2-(3-méthylpipérazine)pyridine. P.f. 209-212°C.
   Analyse élémentaire:
   - calc.: N_{basique} 11,28 %, Clₜₒₜ 28,57 %
   - trouv.: N_{basique} 11,27 %, Clₜₒₜ 29,41 %

## Revendications

1. Utilisation d'au moins un composé de formule (I) 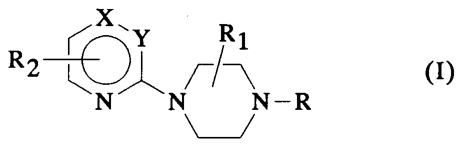 dans laquelle R représente un atome d'hydrogène ou un radical (C₁-C₄)alkyle, R₁ représente un atome d'hydrogène ou un groupe méthyle, R₂ représente un atome d'hydrogène ou d'halogène; X et Y représentent chacun -CH=, ou l'un est -CH= et l'autre -N=, ainsi que de ses sels pharmaceutiquement acceptables pour la préparation de médicaments destinés au traitement et à la prophylaxie des états pathologiques qui peuvent être améliorés à l'aide d'une action agoniste par médiation par les récepteurs du type 5-HT₃.

2. Utilisation selon la revendication 1 d'au moins un composé de formule (I), dans laquelle X et Y sont identiques et représentent chacun -CH=.

3. Utilisation selon la revendication 2 d'au moins un composé de formule (I), dans laquelle R₂ est en position 6 de la pyridine.

4. Utilisation selon la revendication 3 d'au moins un composé de formule (I), dans laquelle R₂ est un atome de chlore ou de brome.

5. Utilisation selon la revendication 4, de la 6-chloro-2-pipérazinylpyridine ou d'un de ses sels pharmaceutiquement acceptables.

6. Utilisation selon la revendication 1 dans le traitement ou la prophylaxie des troubles dysthymiques, de l'anxiété, des troubles psychotiques et des désordres de la motilité intestinale.

7. 6-Chloro-2-(3-méthylpipérazinyl)pyridine et ses sels d'addition pharmaceutiquement acceptables.

8. 6-Chloro-2-(4-méthylpipérazinyl)pyridine et ses sels d'addition pharmaceutiquement acceptables.

9. 6-Bromo-2-(4-méthylpipérazinyl)pyridine et ses sels d'addition pharmaceutiquement acceptables.

10. Compositions pharmaceutiques contenant à titre de principe actif au moins l'un des composés selon l'une quelconque des revendications 7 à 9.
